# EUROPÄISCHE PATENTANMELDUNG

(11) **EP 1 323 741 A2**
(43) Veröffentlichungstag der Anmeldung: **02.07.2003**
(21) Anmeldenummer: 02027469.2
(22) Anmeldetag: 10.12.2002
(51) Int. Cl.: C08F 2/26, C08F 16/26, C07C 59/58

(54) **Olefinisch ungesättigte Ethercarbonsäuren und ihre Verwendung in der Emulsionsspaltung**

(30) Priorität: 21.12.2001 DE 10163258
(71) Anmelder: Clariant GmbH, 65929 Frankfurt am Main (DE)
(72) Erfinder: Falk, Uwe, Dr., 63486 Bruchköbel (DE); Pöllmann, Klaus, Dr., 84489 Burghausen (DE); Ahrens, Hendrik, Dr., 65931 Frankfurt am Main (DE)
(74) Vertreter: Mikulecky, Klaus

(57) **Zusammenfassung**

Verbindungen der Formel (1) 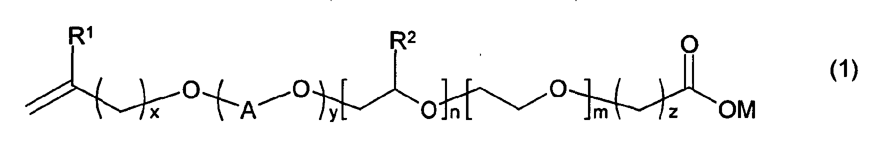 worin
- R¹: H oder C₁-C₄-Alkyl,
- R²: Methyl oder Ethyl,
- A: ein Alkylenrest mit 2 bis 4 C-Atomen,
- x: 0 oder 1
- y: 0 oder 1
mit der Maßgabe, dass (x + y) stets 1 ergibt
- n: eine ganze Zahl von 0 bis 100,
- m: eine ganze Zahl von 0 bis 1000,
mit der Maßgabe, dass (n + m) größer oder gleich 1 ist,
- z: eine ganze Zahl von 1 bis 6, und
- M: Wasserstoff, ein Alkalimetallion oder ein Ammoniumion
bedeuten.

## Beschreibung

Die vorliegende Erfindung betrifft neue Allylpolyalkylenglykolethercarboxylate und Vinylpolyalkylenglykolethercarboxylate sowie ihre Verwendung als copolymerisierbare Emulgatoren und hydrophile Monomere in der Emulsionspolymerisation.

Bei der Emulsionspolymerisation werden olefinisch ungesättigte und wasserunlösliche Monomere durch Emulgatoren im wässrigen Medium in Form von Mizellen dispergiert. Radikalbildner initiieren dann die Polymerisationsreaktion, die in den in situ gebildeten Polymerpartikeln stattfindet.

Die für die Emulsionspolymerisation nach dem Stand der Technik eingesetzten Emulgatoren sind zumeist anionische und nichtionische Emulgatoren. Diese Emulgatoren sind über physikalische Kräfte an die Oberfläche der Polymerpartikel gebunden.

Übliche anionische Emulgatoren sind Natrium-, Kalium- und Ammoniumsalze von Fettsäuren, Natriumalkylbenzolsulfonate, Natriumalkylsulfonate, Natriumolefinsulfonate, Natriumpolynaphthalensulfonate, Natriumdialkyldiphenyletherdisulfonate, Natrium-, Kalium- und Ammoniumalkylsulfate, Natrium-, Kalium- und Ammoniumalkylpolyethylenglykolethersulfate, Natrium-, Kalium- und Ammoniumalkylphenolpolyethylenglykolethersulfate, Natrium-, Kalium- und Ammoniummono- und dialkylsulfosuccinate und monoalkylpolyoxethylsulfosuccinate, sowie Alkylpolyethylenglykoletherphosphorsäuremono-, di- und triester und deren Mischungen und Alkylphenolpolyethylenglykoletherphosphorsäuremono-, di- und triester und deren Mischungen, sowie deren Natrium-, Kalium- und Ammoniumsalze.

Als nichtionische Emulgatoren werden üblicherweise Alkylphenolpolyethylenglykolether, Alkylpolyethylenglykolether, Fettsäurepolyethylenglykolether, Ethylen/Propylenglykol-Blockpolymere und Sorbitanesterpolyethylenglykolether eingesetzt.

Die sich in situ bildenden Polymerpartikel werden durch die eingesetzten Emulgatoren stabilisiert und am Koagulieren gehindert.

Eine Übersicht über gängige Verfahren, Tenside und weitere Hilfsmittel der Emulsionspolymerisation geben Peter A. Lovell und Mohamed S. EI-Aasser, in "Emulsion Polymerization and Emulsion Polymers", erschienen bei John Wiley and Sons, 1997.

Neben den anionischen und nichtionischen Tensiden können während der Polymerisation auch hydrophile, wasserlösliche und olefinisch ungesättigte Monomere, die über mindestens eine anionische Gruppe im Molekül verfügen, zur Stabilisierung der Polymerpartikel eingesetzt werden. Zu diesen hydrophilen, wasserlöslichen Monomere gehören beispielsweise:
- olefinisch ungesättigte Carbonsäuren, wie Acrylsäure, Methacrylsäure, Crotonsäure und Itaconsäure und ihre Natrium-, Kalium- und Ammoniumsalze,
- olefinisch ungesättigte Polycarbonsäuren, wie Maleinsäure und Fumarsäure und ihre Natrium-, Kalium- und Ammoniumsalze
- olefinisch ungesättigte Sulfonsäuren und ihre Alkali- und Ammoniumsalze, wie Acrylamidomethylpropansulfonsäure und ihre Alkali- und Ammonium-, Alkylammonium- und Hydroxyalkylammoniumsalze, Allyl- und Vinylsulfonsäure und ihre Alkali- und Ammoniumsalze,
- olefinisch ungesättigte Phosphonsäuren und ihre Alkali- und Ammonium-, Alkylammonium- und Hydroxyalkylammoniumsalze, wie Allylphosphonsäure, Vinylphosphonsäure, Acryloyloxethylphosphonsäure und ihre Ammoniumund Alkalisalze sowie die entsprechenden Methacrylsäurederivate.

Wasserunlösliche Monomere sind beispielsweise folgende:
- Vinylmonomere wie Carbonsäureester des Vinylalkohols, beispielsweise Vinylacetat, Vinylpropionat, Vinylether der Isononansäure oder der Isodecansäure, Styrol und Stilben,
- olefinisch ungesättigte Carbonsäureester, wie Ethylacrylat, n-Butylacrylat, i-Butylacrylat, Hexylacrylat, 2-Ethylhexylacrylat, Hydroxyethylacrylat sowie die entsprechenden Methacrylsäureester,
- olefinisch ungesättigte Amine, Ammoniumsalze, Nitrile und Amide, wie Dimethylaminoethylacrylat, Acryloyloxethyltrimethylammoniumhalide, Acylnitril, N-Methacrylamid, N-Ethylacrylamid, N-Propylacrylamid sowie die entsprechenden Methacylsäurederivate und Vinylmethylacetamid,
- Olefine, wie Ethylen, Propen und Butene, Penten, 1,3-Butadien und Chloropren,
- Vinylhalogenide, wie Vinylchlorid, Vinylidenchlorid und Vinylidenfluorid.

Die hydrophilen, wasserlöslichen Monomere reagieren mit den wasserunlöslichen Monomeren, so dass die in das Polymerpartikel eingebauten anionischen Gruppen aufgrund von elektrostatischen Wechselwirkungen zur Stabilisierung der Polymerpartikel beitragen. Nachteil der hydrophilen, wasserlöslichen Monomere mit einer oder mehreren anionischen Gruppen ist ihre gute Wasserlöslichkeit, da sie sich dadurch hauptsächlich in der wässrigen Phase befinden und sich nicht an den Polymerpartikeln anlagern, in denen die Polymerisationsreaktion stattfindet, sondern vielmehr durch radikalische Homopolymerisation in der wässrigen Phase gut wasserlöslich Oligomere bilden, die nicht zur Stabilisierung der Polymerpartikel beitragen.

Aufgabe vorliegender Erfindung war es demzufolge, neue copolymerisierbare Emulgatoren zu finden, die in der Emulsionspolymerisation verwendbar sind. Diese Emulgatoren sollen die oben beschriebenen Nachteile der hydrophilen Monomere bei der Stabilisierung der Polymerpartikel nicht aufweisen. Es soll mit ihnen möglich sein, auf die stabilisierende Wirkung hydrophiler Monomere zu verzichten.

Es wurden nun neue Allyl- und Vinylpolyalkylenglycolethercarboxylate gefunden, die nach der Williamson-Synthese durch Umsetzung von Allyl- und Vinylpolyalkylenglykolethern mit Monochloralkancarbonsäuren hergestellt werden können. Aufgrund ihres ambivalenten Charakters lagern sich die neuen Allyl- und Vinylpolyalkylenglycolethercarboxylate an der Oberfläche der Polymerpartikel an und können mit den olefinisch ungesättigten und wasserunlöslichen Monomere reagieren und chemisch in die Polymerpartikel eingebunden werden. Mit den neuen Allyl- und Vinylpolyalkylenglycolethercarboxylate lassen sich stabile und koagulatarme Polymerdispersionen herstellt.

Gegenstand der Erfindung sind Verbindungen der Formel (1) 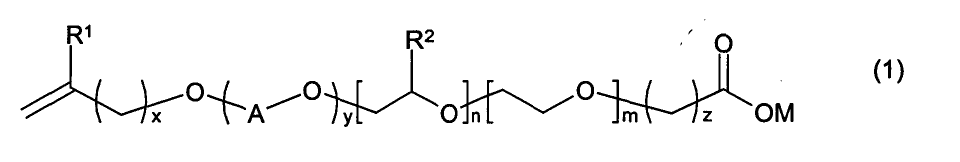 worin
- R¹: H oder C₁-C₄-Alkyl,
- R²: Methyl oder Ethyl,
- A: ein Alkylenrest mit 2 bis 4 C-Atomen,
- x: 0 oder 1
- y: 0 oder 1
mit der Maßgabe, dass (x + y) stets 1 ergibt
- n: eine ganze Zahl von 0 bis 100,
- m: eine ganze Zahl von 0 bis 1000,
mit der Maßgabe, dass (n + m) größer oder gleich 1 ist,
- z: eine ganze Zahl von 1 bis 6, und
- M: Wasserstoff, ein Alkalimetallion oder ein Ammoniumion
bedeuten.

Ein weiterer Gegenstand der Erfindung ist die Verwendung der erfindungsgemäßen Verbindungen als polymerisierbare Emulgatoren und hydrophile Monomere in der Emulsionspolymerisation.

Ein weiterer Gegenstand der Erfindung ist ein Verfahren zur Emulsionspolymerisation, in dem Verbindungen der Formel 1 zusammen mit einem oder mehreren olefinisch ungesättigten Monomeren der radikalischen Polymerisation unterzogen werden.

Die Verbindungen der Formel 1 können allein in Suspensions- oder Emulsionspolymerisationen als Emulgator verwendet werden, sowie in Mischung mit anionischen und/oder nichtionischen Tensiden, die im Stand der Technik bekannt sind.

Die Struktureinheiten 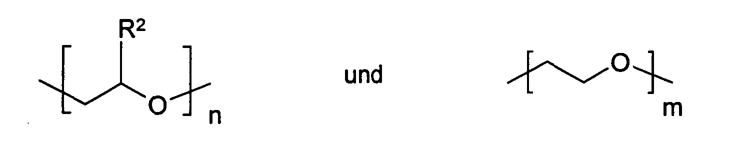 aus Formel (1) können als Blöcke in der in Formel (1) angegebenen Reihenfolge, als Blöcke in gegenüber Formel (1) vertauschter Reihenfolge, sowie in statistischer Abfolge (random) vorliegen. Bevorzugt sind Blöcke.

R¹ steht vorzugsweise für einen Methyl- oder Ethylrest oder Wasserstoff, insbesondere für Wasserstoff.

R² steht vorzugsweise für eine Methylgruppe.

A steht vorzugsweise für eine Ethylen-, Butylen- oder Propylengruppe, insbesondere für eine Ethylengruppe oder Butylengruppe.

n steht vorzugsweise für eine Zahl von 2 bis 50, insbesondere von 3 bis 30.

m steht vorzugsweise für eine Zahl von 2 bis 200, insbesondere von 3 bis 100.

z steht vorzugsweise für eine Zahl von 2 bis 4.

Die erfindungsgemäßen Allyl- und Vinylpolyalkylenglykolethercarboxylate können im Reaktionsgefäß vor Beginn der Polymerisationsreaktion vorgelegt oder während der Polymerisationsreaktion dem Reaktionsgefäß zugegeben werden.

Im allgemeinen werden die erfindungsgemäßen Verbindungen der Formel (1) in Mengen von 0,1 bis 50, vorzugsweise 0,2 bis 10, insbesondere 0,4 bis 4 Gew.-%, bezogen auf das Gewicht der für die Herstellung der Polymerdispersion verwendeten nicht oder wenig wasserlöslichen Monomere als Emulgatoren und hydrophile Monomere verwendet.

Die erfindungsgemäßen Verbindungen eignen sich zur Herstellung von stabilen Polymerdispersionen. Zur Herstellung dieser Polymerdispersionen sind ungesättigte Monomere geeignet, mit denen die erfindungsgemäßen Verbindungen polymerisiert werden. Geeignete Monomere sind solche Verbindungen, die wenigstens eine olefinische Doppelbindung aufweisen, welche mit Wasserstoff oder einem oder mehreren Resten substituiert ist, wobei diese Reste Kohlenwasserstoffreste oder Heteroatome tragende Kohlenwasserstoffreste sein können, die 1 bis 50 Kohlenstoffatome umfassen. Bevorzugte olefinisch ungesättigte Monomere sind beispielsweise
- Vinylmonomere, wie Carbonsäureester des Vinylalkohols, beispielsweise Vinylacetat, Vinylpropionat, Vinylether der Isononansäure oder der Isodecansäure,
- Arylsubstituierte Olefine, wie Styrol und Stilben
- olefinisch ungesättigte Carbonsäureester, wie Methylacrylat, Ethylacrylat, Propylacrylat, n-Butylacrylat, i-Butylacrylat, Pentylacrylat, Hexylacrylat, 2-Ethylhexylacrylat, Hydroxyethylacrylat sowie die entsprechenden Methacrylsäureester,
- olefinisch ungesättigte Dicarbonsäureester, wie Dimethylmaleinat, Diethylmaleinat, Dipropylmaleinat, Dibutylmaleinat, Dipentylmaleinat, Dihexylmaleinat und Di-2-ethyihexyimaieinat,
- olefinisch ungesättigte Carbonsäuren und Dicarbonsäuren, wie Acrylsäure, Methacrylsäure, Itaconsäure, Maleinsäure und Fumarsäure und ihre Natrium-, Kalium- und Ammoniumsalze,
- olefinisch ungesättigte Sulfonsäuren und Phosphonsäuren und ihre Alkaliund Ammoniumsalze, wie Acrylamidomethylpropansulfonsäure und ihre Alkali- und Ammonium-, Alkylammonium und Hydroxyalkylammoniumsalze, Allylsulfonsäure und ihre Alkali- und Ammoniumsalze, Acryloyloxethylphosphonsäure und ihre Ammonium- und Alkalisalze sowie die entsprechenden Methacrylsäurederivate,
- olefinisch ungesättigte Amine, Ammoniumsalze, Nitrile und Amide, wie Dimethylaminoethylacrylat, Acryloyloxethyltrimethylammoniumhalide, Acrylnitril, N-Methylacrylamid, N-Ethylacrylamid, N-Propylacrylamid, N-Methylolacrylamid sowie die entsprechenden Methacrylsäurederivate und Vinylmethylacetamid.

In einer bevorzugten Ausführungsform werden die oben genannten Monomere mit weiteren Comonomeren, vorzugsweise Olefinen oder halogenierten Olefinen mit 2 bis 8 Kohlenstoffatomen wie z.B. Ethylen, Propen, Butene, Pentene, 1,3-Butadien, Chloropren, Vinylchlorid, Vinylidenchlorid, Vinylidenfluorid und Tetrafluorethylen polymerisiert.

Die erfindungsgemäßen Verbindungen können sowohl alleine als auch in Kombination mit anderen bereits bekannten anionischen und nichtionischen Emulgatoren des Standes der Technik verwendet werden, wie sie eingangs beschrieben wurden. Die Menge der anionischen und nichtionischen Emulgatoren des Standes der Technik beträgt dann vorzugsweise 0,001 bis 5, insbesondere 0,01 bis 1 und besonders bevorzugt 0,02 bis 0,5 Gew.-% bezogen auf das Gewicht der nicht oder wenig wasserlöslichen Monomere.

Weitere Hilfs- und Zusatzstoffe für die Verwendung mit den erfindungsgemäßen Verbindungen können Schutzkolloide wie Carboxymethylcellulose, Hydroxyethylcellulose, Methylhydroxypropylcellulose, sowie teil- und vollverseifter Polyvinylalkohol sein.

Die mit den erfindungsgemäßen neuen Allyl- und Vinylpolyalkylenglykolethercarboxylaten hergestellten Polymerdispersionen eignen sich für die Anwendung als Beschichtungsmittel textiler Flächengebilde, als Papierstrich in der Papier- und Kartonagenherstellung, als Bindemittel für Pigmente und Füllstoffe in Druckpasten für Textilien, Leder, Papiere, Kartonagen, sowie für Beschichtungen und Anstrichfarben für mineralische Oberflächen, Holz, Metalle und Kunststoffe und als Klebrohstoff in Klebstoffformulierungen.

Folgende Beispiele verdeutlichen die Erfindung näher.

### Beispiel 1

Es wurden 730 g Allylpolyalkylenglykolether aus 4 Mol Propylenoxid und 10 Mol Ethylenoxid unter Stickstoffatmosphäre auf 50°C erwärmt und 160 g Monochloressigsäure innerhalb von 10 Minuten zugegeben. Zu der Reaktionsmischung wurden unter Rühren in 8 Portionen innerhalb von 2 Stunden bei 50°C 62 g Natriumhydroxid-Prills zugegeben. Anschließend wurde das Reaktionsgemisch auf 70°C erwärmt und 2 Stunden temperiert. Nach dem Abkühlen wurden 952 g des Natriumallylpolyalkylenglykolethercarboxylat erhalten.

### Beispiel 2

Es wurden 473 g Vinylpolyalkylenglykolether, einem Additionsprodukt aus 4-Hydroxybutylvinylether mit 4 Mol Propylenoxid und 10 Mol Ethylenoxid, unter Stickstoffatmosphäre auf 50°C erwärmt und 96 g Monochloressigsäure innerhalb von 10 Minuten zugegeben. Zu der Reaktionsmischung wurden unter Rühren in 8 Portionen innerhalb von 2 Stunden bei 50°C 37 g Natriumhydroxid-Prills zugegeben. Anschließend wurde das Reaktionsgemisch auf 70°C erwärmt und 2 Stunden temperiert. Nach dem Abkühlen wurden 606 g des Natriumsalzes der Vinylpolyalkylenglykolethercarbonsäure erhalten.

### Beispiel 3

Das Makromonomer aus Beispiel 2 wurde als Coemulgator bei der Emulsionspolymerisation von Styrol, n-Butylacrylat, Methylmethacylat und Methacylsäure verwendet. Das sich in situ bildende Copolymer aus Styrol, n-Butylacrylat, Methylmethacylat und Methacylsäure und dem Makromonomer aus Beispiel 2 hat auf Basis seiner ambivalenten Struktur gute emulsionsstabilisierende Eigenschaften.

358 ml Wasser wurden in einem Glaskolben vorgelegt und 8 g ®Emulsogen EPA 073 (Natriumalkylethersulfat), 37 g einer 3,75%igen Ammoniumperoxodisulfat-Lösung, 9 g Styrol, 12,5 g n-Butylacrylat, 6 g Methylmethacylat und 0,3 g Methacrylsäure zugegeben und gerührt. Unter Rühren wurde die Emulsion auf 80°C geheizt und über 3 Stunden eine Monomeremulsion zudosiert, die aus 625 ml Wasser, 28 g Emulsogen EPA 073, 22 g des Makromonomers aus Beispiel 2, 350 g Styrol, 512 g n-Butylacrylat, 234 g Methylmethacrylat, 10,7 g Methacrylsäure und 4,2 g Ammoniumperoxodisulfat bestand. Nach vollständiger Dosierung der Monomeremulsion und einer Nachpolymerisation von einer Stunde bei 80°C wurde die Polymerdispersion auf Raumtemperatur abgekühlt und mit Ammoniak―Lösung auf pH 7 - 8 eingestellt.

### Beispiel 4

Das Makromonomer aus Beispiel 1 wurde als Coemulgator bei der Emulsionspolymerisation von n-Butylacrylat, Methylmethacylat und Methacylsäure verwendet. Das sich in situ bildende Copolymer aus n-Butylacrylat, Methylmethacylat und Methacylsäure und dem Makromonomer aus Beispiel 1 hat auf Basis seiner ambivalenten Struktur gute emulsionsstabilisierende Eigenschaften.

520 ml Wasser wurden in einem Glaskolben vorgelegt und 16 g ®Emulsogen EPA 073 (Natriumalkylethersulfat), 15 g einer 3,75%igen Ammoniumperoxodisulfat-Lösung, 11,8 g n-Butylacrylat, 11,8 g Methylmethacylat und 0,5 g Methacrylsäure zugegeben und gerührt. Unter Rühren wurde die Emulsion auf 80°C geheizt und über 4 Stunden eine Monomeremulsion zudosiert, die aus 460 ml Wasser, 32 g Emulsogen EPA 073, 12 g des beschriebenen Macromonomers aus Beispiel 1, 440 g n-Butylacrylat, 440 g Methylmethacrylat, 8,8 g Methacrylsäure und 2,9 g Ammoniumperoxodisulfat bestand. Nach vollständiger Dosierung der Monomeremulsion und einer Nachpolymerisation von einer Stunde bei 80°C wurde die Polymerdispersion auf Raumtemperatur abgekühlt und mit Ammoniak-Lösung auf pH 8 ― 9 eingestellt.

### Beispiel 5

Das Makromonomer aus Beispiel 2 wurde als Coemulgator bei der Emulsionspolymerisation von Vinylacetat, n-Butylacrylat und Methacylsäure verwendet. Das sich in situ bildende Copolymer aus Vinylacetat, n-Butylacrylat und Methacylsäure und dem Makromonomer aus Beispiel 2 hat auf Basis seiner ambivalenten Struktur gute emulsionsstabilisierende Eigenschaften.

508 ml Wasser wurden in einem Glaskolben vorgelegt und 0,1 g ®Emulsogen EP (Natriumalkylsulfonat), 0,2 g Natriummetabisulfit, 31 g einer 3,75 %igen Ammoniumperoxodisulfat-Lösung, 22 g Vinylacetat, 6 g n-Butylacrylat und 0,3 g Methacrylsäure zugegeben und gerührt. Unter Rühren wurde die Emulsion auf 80°C geheizt und über 3 Stunden eine Monomeremulsion zudosiert, die aus 413 ml Wasser, 3,6 g Emulsogen EP, 27 g des beschriebenen Macromonomers aus Beispiel 2, 858 g Vinylacetat, 214 g n-Butylacrylat, 10,7 g Methacrylsäure und 2,7 g Ammoniumperoxodisulfat bestand. Die Dosierung der Monomeremulsion wurde schrittweise erhöht, so dass nach den ersten 30 Minuten 10% der Monomeremulsion und in den folgenden 150 Minuten die restlichen 90 % der Monomeremulsion zugegeben wurden. Nach vollständiger Dosierung der Monomeremulsion und einer Nachpolymerisation von einer Stunde bei 80°C wurde die Polymerdispersion auf Raumtemperatur abgekühlt.

## Patentansprüche

1. Verbindungen der Formel (1) 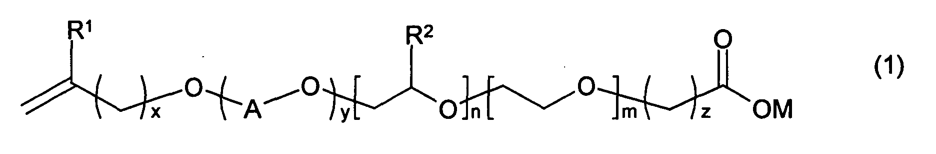 worin
R¹ H oder C₁-C₄-Alkyl,
R² Methyl oder Ethyl,
A ein Alkylenrest mit 2 bis 4 C-Atomen,
x 0 oder 1
y 0 oder 1
mit der Maßgabe, dass (x + y) stets 1 ergibt
n eine ganze Zahl von 0 bis 100,
m eine ganze Zahl von 0 bis 1000,
mit der Maßgabe, dass (n + m) größer oder gleich 1 ist,
z eine ganze Zahl von 1 bis 6, und
M Wasserstoff, ein Alkalimetallion oder ein Ammoniumion
bedeuten.

2. Verbindungen nach Anspruch 1, worin R¹ für einen Methyl- oder Ethylrest oder Wasserstoff steht.

3. Verbindungen nach Anspruch 1 und/oder 2, worin R² für eine Methylgruppe steht.

4. Verbindungen nach einem oder mehreren der Ansprüche 1 bis 3, worin A für eine Ethylen- oder Butylengruppe steht.

5. Verbindungen nach einem oder mehreren der Ansprüche 1 bis 4, worin n für eine Zahl von 2 bis 50 steht.

6. Verbindungen nach einem oder mehreren der Ansprüche 1 bis 5, worin m für eine Zahl von 2 bis 200 steht.

7. Verbindungen nach einem oder mehreren der Ansprüche 1 bis 6, worin z für eine Zahl von 2 bis 4 steht.

8. Verwendung von Verbindungen gemäß einem oder mehreren der Ansprüche 1 bis 7 als polymerisierbare Emulgatoren und hydrophile Monomere in der Emulsionspolymerisation.
